# EUROPEAN PATENT APPLICATION

(11) **EP 4 545 637 A1**
(43) Date of publication of application: **30.04.2025**
(21) Application number: 23827518.4
(22) Date of filing: 21.06.2023
(51) Int. Cl.: C12N 9/26, C07K 14/47

(54) **N-TERMINAL AND/OR C-TERMINAL CLEAVED SOLUBLE PH20 POLYPEPTIDE AND USE THEREOF**

(30) Priority: 22.06.2022 KR 20220076030
(71) Applicant: Alteogen, Inc., Daejeon 34054 (KR)
(72) Inventor: PARK, Soon Jae, Daejeon 34054 (KR); KIM, Kyuwan, Daejeon 34054 (KR); YUN, Sang Hoon, Daejeon 34054 (KR); SONG, Hyung-Nam, Daejeon 34054 (KR)
(74) Representative: Mewburn Ellis LLP
(86) International application number: PCT/KR2023/008621
(87) International publication number: WO 2023/249408

(57) **Abstract**

The present invention relates to a recombinant PH20 polypeptide in which 1 to 7 amino acid residues are deleted at the N-terminus of a mature animal wild-type PH20, and use thereof.

The N-terminal and/or C-terminal cleaved PH20 polypeptide with improved enzymatic activity and productivity presented in the present invention may exhibit superior expression and enzymatic activity compared to conventional recombinant PH20 polypeptides, which may result in reduced production costs for industrial use and thus reduced treatment costs.

## Description

### Technical Field

The present invention relates to an N-terminal and/or C-terminal cleaved soluble PH20 polypeptide and use thereof.

### Background Art

There have been constant attempts, by using genetic engineering methods, to increase the expression level of industrially useful proteins secreted from recombinant cells.

Several conventional methods have been tried, and each method has its own advantages and disadvantages. For example, the proper promoters were chosen in the cloning step to increase the expression level, however, when the protein folding is rate limiting step, the amount of expressed protein were not much enough which was expected from chosen promoter. (Su Xiao et al., Curr Opin Struct Biol. 2014, June 32-38*).*

In order to increase the expression of proteins that are secreted from the cell, signal peptides of target protein at N-terminus has been substituted to signal peptide from other proteins. (Kober, L., Zehe, C. & Bode, J., 2013. Optimized Signal Peptides for the Development of High Expressing CHO Cell Lines. Biotechnology and bioengineering, 110(4), pp. 1164-1173). In this case, protein expression sometimes increases compared to the case of using its own signal peptide, but when the expressed protein is cleaved by the signal peptidase while passing through the endoplasmic reticulum membrane in the cell, signal peptidase sometimes fails to cleave precisely between the signal peptide and the N-terminal amino acid of mature protein.

Other attempts to increase protein expression include coexpressing with a chaperon to help folding of a recombinant protein, and prevent aggregation of proteins to be expressed. However, this method is not often used industrially due to the technical challenge which is to express target protein and membrane bound chaperone at the same time in recombinant cell and the intracellular physiological challenge caused by chaperone expression.

Another attempt to increase protein expression is amino acid substitution of recombinant protein by using site directed mutagenesis at specific site. A protein composed of polypeptides folds into three-dimensional structure when produced in the cell. Amino acid substitution can provide favorable environment for protein folding which can lead an increase in protein expression. There have been many successful cases, but practical problems such as increase in immunogenicity due to amino acid modification are inherent.

Hyaluronan (hyaluronic acid: HA) is a glycosaminoglycan found in the extracellular matrix of many cells, particularly in the connective tissues. Hyaluronan is also found primarily in mammalian skin, cartilage and synovial fluid. Hyaluronan is also a major component of the vitreous humor of an eye. Hyaluronan plays a role in various physiological processes, such as the homeostasis of water and plasma proteins (Laurent TC et al (1992) FASEB J 6: 2397-2404). Certain diseases are associated with expression and/or production of hyaluronan. Hyaluronidase is an enzyme that breaks down hyaluronan. Hyaluronidase can be used to treat diseases or disorders associated with accumulation of hyaluronan or other glycosaminoglycan by catalyzing hydrolysis of hyaluronan. In addition, because hyaluronan is a major component of the subcutaneous skin barrier, hyaluronidase may be used to increase tissue permeability and, therefore, to increase dispersion and delivery of therapeutic agents when injected subcutaneously.

Various hyaluronidases based on native PH20 polypeptide (e.g., Hydase^{™}, Vitrase^{™}, Wydase^{™}) have been used in combination with other therapeutic agents, typically as dispersing agents. Many of these hyaluronidases comprise PH20 derived from ovine or bovine testicles.

Human PH20 protein consists of 509 amino acids, and is known as a glycophosphoinositol lipid anchored protein present in the plasma membrane of sperm. While hyaluronidases Hyal1, Hyal2, Hyla3, or Hyal4 present in blood are only active at acidic pH, PH20 is active even at neutral pH, so PH20 has been developed to be mixed with drugs for subcutaneous injection, and has been industrially used.

Recently, Hylenex^{™}, a 447 amino acids recombinant human PH20 polypeptide modified to be soluble by cleaving amino acids at the C-terminus of PH20 which are responsible for anchoring glycophosphoinositol has been used. In addition, materials using a variant of human PH20 polypeptide are also being developed.

Meanwhile, a human PH20, a glycoprotein that is N-linked glycosylated at six sites, has a very complex three-dimensional structure, and, to produce an industrially useful enzyme, requires a complex purification process after expression in animal cells such as CHO cells. Therefore, for industrial utilization of human PH20, it is very important to improve productivity of animal cells containing human PH20 genes depending on the expression level thereof in a fermentation media.

Under this technical background, the inventors of the present invention have identified that cleavage of N-terminus or N- and C-termini can improve productivity of soluble recombinant PH20 polypeptide, and have completed the present invention.

### Summary of the Invention

The present invention aims to provide an N-terminal and/or C-terminal cleaved soluble PH20 polypeptide with improved productivity.

The present invention aims to provide a nucleic acid encoding the N-terminal and/or C-terminal cleaved soluble PH20 polypeptide.

The present invention aims to provide a recombinant expression vector comprising the nucleic acid.

The present invention aims to provide a host cell transformed with the recombinant expression vector.

The present invention relates to a method of producing the N-terminal and/or C-terminal cleaved soluble PH20 polypeptide, comprising the step of culturing the host cell.

The present invention provides a recombinant PH20 polypeptide in which 1 to 7 amino acid residues are deleted from the N-terminus of mature animal wild-type PH20.

Specifically, a recombinant PH20 polypeptide in which amino acid residue(s) is(are) deleted from the N-terminus of mature animal wild-type PH20 according to the present invention can be characterized as selected from the group consisting of, but is not limited to:
(a) a recombinant PH20 polypeptide in which (an) N-terminal amino acid residue(s) is(are) deleted by cleavage before an amino acid residue selected from the group consisting of N37 to P42 in human wild-type PH20 having the amino acid sequence of SEQ ID No. 1;
(b) a recombinant PH20 polypeptide in which (an) N-terminal amino acid residue(s) is(are) deleted by cleavage before an amino acid residue selected from the group consisting of N37 to P42 in primate wild-type PH20 having the amino acid sequence of any one of SEQ ID Nos. 2 to 8;
(c) a recombinant PH20 polypeptide in which (an) N-terminal amino acid residue(s) is(are) deleted by cleavage before an amino acid residue selected from the group consisting of D37 to P42 in bovine wild-type PH20 having the amino acid sequence of SEQ ID No. 9; and
(d) a recombinant PH20 polypeptide in which (an) N-terminal amino acid residue(s) is(are) deleted by cleavage before an amino acid residue selected from the group consisting of D37 to P42 in ovine wild-type PH20 having the amino acid sequence of SEQ ID No. 10.

The present invention provides a nucleic acid encoding the recombinant PH20 polypeptide and a recombinant expression vector comprising the nucleic acid.

The invention also provides a host cell transformed with the recombinant expression vector, and a method of producing an N-terminal and/or C-terminal cleaved soluble PH20 polypeptide, comprising the step of culturing the host cell.

### Description of Drawing

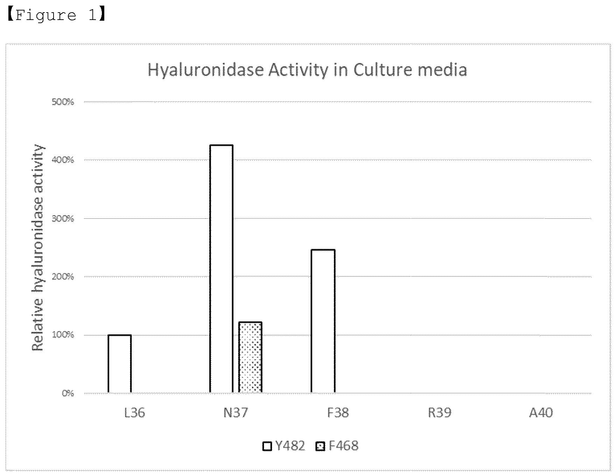

FIG. 1 is a diagram showing the enzymatic activities in cultures of PH20 polypeptides in which the C-terminus of SEQ ID No. 1 is Y482 or F468, and the N-terminus begins with L36, N37, F38, R39 or A40, according to the embodiments of the present invention. Each activity is expressed as a relative activity percentage based on L36-Y482 having the longest amino acid length.

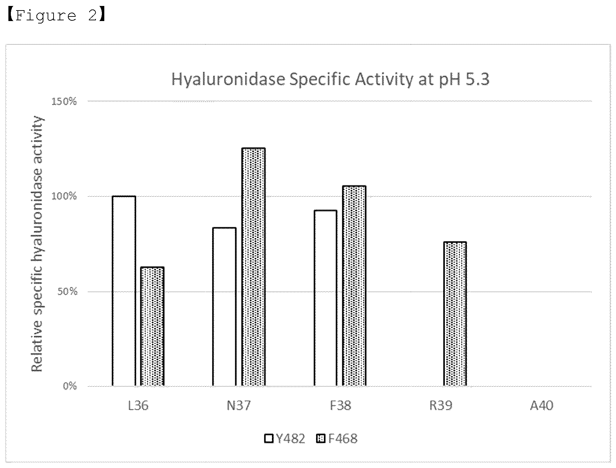

FIG. 2 is a diagram showing the enzymatic activities, expressed as specific activities, at pH 5.3 of purified PH20 polypeptides in which the C-terminus of SEQ ID No. 1 is Y482 or F468, and the N-terminus begins with L36, N37, F38, R39 or A40, according to the embodiments of the present invention. Each activity is expressed as a relative activity percentage based on L36-Y482 having the longest amino acid length.

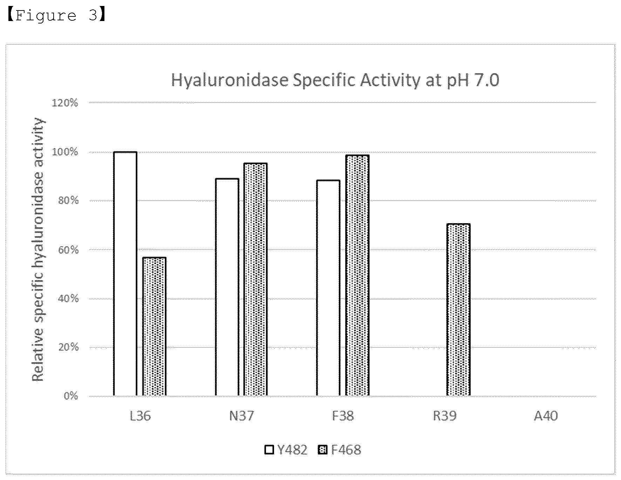

FIG. 3 is a diagram showing the enzymatic activities, expressed as specific activities, at pH 7.0 of purified PH20 polypeptides in which the C-terminus of SEQ ID No. 1 is Y482 or F468, and the N-terminus begins with L36, N37, F38, R39 or A40, according to the embodiments of the present invention. Each activity is expressed as a relative activity percentage based on L36-Y482 having the longest amino acid length.

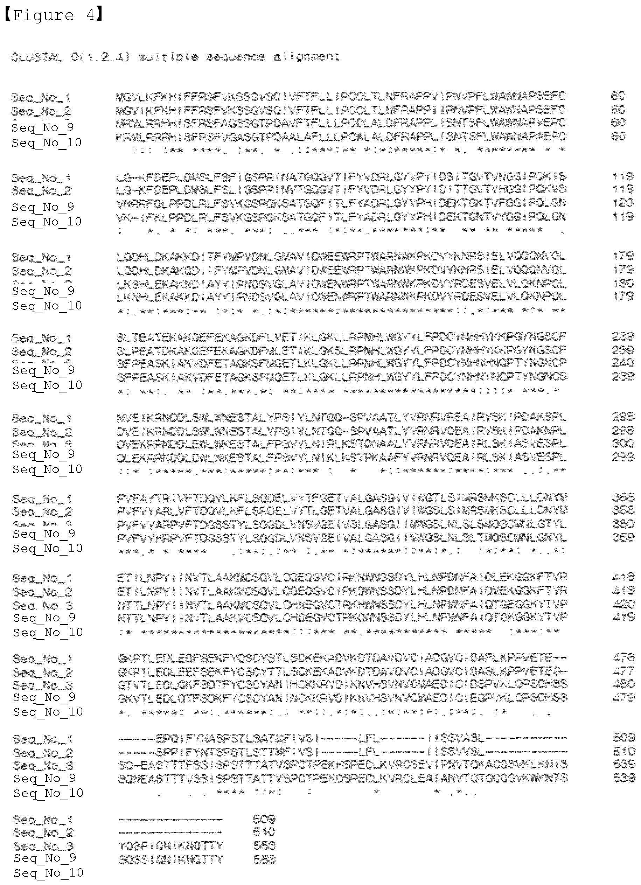

FIG. 4 shows the results of multiple sequence alignment using Clustal Omega (https://www.ebi.ac.uk/Tools/msa/clustalo/) of the amino acid sequences of SEQ ID Nos. 1, 2, 9 and 10.

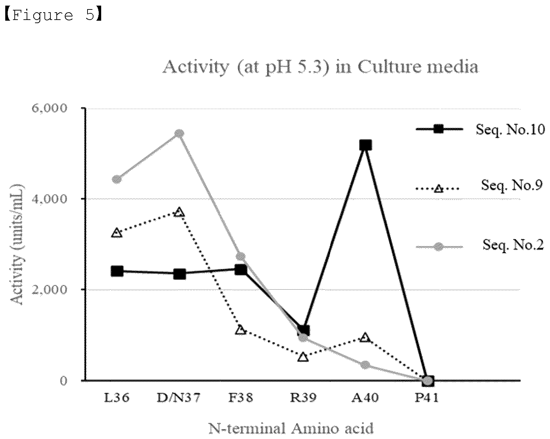

FIG. 5 is a diagram showing the enzymatic activities of culture media of PH20 polypeptides whose N-terminus starts with L36, N37 or D37, F38, R39, A40 or P41 for SEQ ID No. 2 (in which C-terminus is Y483), SEQ ID No. 9 (in which C-terminus is H478) or SEQ ID No. 10 (in which C-terminus is H477), respectively, according to the embodiments of the present invention. Each activity is expressed as a measured value.

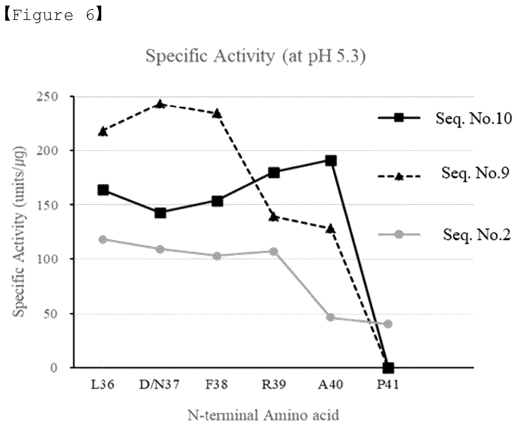

FIG. 6 is a diagram showing the enzymatic activities, expressed as specific activities, at pH 5.3 of purified PH20 polypeptides whose N-terminus starts with L36, N37, F38, R39, A40 or P41 for SEQ ID No. 2 (in which C-terminus is Y483), SEQ ID No. 9 (in which C-terminus is H478) or SEQ ID No. 10 (in which C-terminus is H477), respectively, according to the embodiments of the present invention. Each activity is expressed as a measured value.

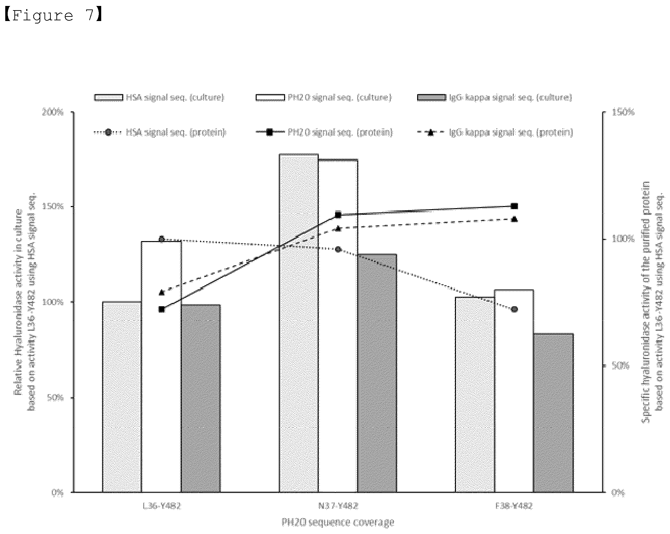

FIG. 7 shows the results of measuring the enzymatic activities at pH 5.3 of PH20 polypeptides in the cultures and in the purified state having different signal peptides for L36-Y482, N37-482 and F38-Y482 of SEQ ID No. 1, according to the embodiments of the present invention. The enzymatic activities of the culture and the purified protein using a signal peptide from human serum albumin, the enzymatic activities of the culture and the purified protein using a signal peptide from human PH20, and the enzymatic activities of the culture and the purified protein using a signal peptide from human immunoglobulin kappa are presented in FIG. 7. The enzyme activity scale of the culture is presented on the main y-axis as a bar graph, and the activity scale of the purified protein is presented on the auxiliary y-axis as a line graph.

### Detailed Description of the Present Invention and Preferred Embodiments

Unless otherwise defined, all technical and scientific terms used herein have the same meanings as commonly understood by a person skilled in the art under which the present invention falls. In general, the nomenclature used herein is well known and commonly used in the art.

In order to expand the industrial applicability of a recombinant PH20 polypeptide that is cleaved at the C-terminus of PH20 and converted to soluble form, the present invention provides an N-terminal and/or C-terminal cleaved PH20 polypeptide with significantly improved productivity by further cleaving the N-terminus of PH20. The term "soluble" used herein refers to a form having a three-dimensional structure that is active in an aqueous solution without aggregation.

Accordingly, the present invention relates to a recombinant PH20 polypeptide in which 1 to 7 amino acid residues, preferably 1 to 6 amino acid residues, preferably 1 to 5 amino acid residues, preferably 1 to 4 amino acid residues, preferably 1 to 3 amino acid residues, preferably 1 to 2 amino acid residues, preferably 1 amino acid residue is deleted from the N-terminus of mature animal wild-type PH20.

The mature animal wild-type PH20 in the present invention may refer a recombinant PH20 polypeptide in a form that exhibits the desired function. The mature animal wild-type PH20 according to the present invention may refer, for example, a state in which a signal peptide that promotes secretion of mature animal wild-type PH20 outside the cell has been cleaved during the secretion process.

The animal may be, for example, but not limited to, a mammal, rodent, or the like, such as a human, rat, mouse, hamster, rabbit, pig, cow, deer, sheep, monkey, or the like.

The animal wild-type PH20 according to the present invention may include, for example, but is not limited to:
(a) a recombinant PH20 polypeptide in which (an) N-terminal amino acid residue(s) is(are) deleted by cleavage before an amino acid residue selected from the group consisting of N37 to P42 in human wild-type PH20 having the amino acid sequence of SEQ ID No. 1;
(b) a recombinant PH20 polypeptide in which (an) N-terminal amino acid residue(s) is(are) deleted by cleavage before an amino acid residue selected from the group consisting of N37 to P42 in primate wild-type PH20 having the amino acid sequence of any one of SEQ ID Nos. 2 to 8;
(c) a recombinant PH20 polypeptide in which (an) N-terminal amino acid residue(s) is(are) deleted by cleavage before an amino acid residue selected from the group consisting of D37 to P42 in bovine wild-type PH20 having the amino acid sequence of SEQ ID No. 9; and
(d) a recombinant PH20 polypeptide in which (an) N-terminal amino acid residue(s) is(are) deleted by cleavage before an amino acid residue selected from the group consisting of D37 to P42 in ovine wild-type PH20 having the amino acid sequence of SEQ ID No. 10.

**Table 1. Information of amino acid sequences of animal PH20**

| **Animal name** | **Scientific Name** | Uniprot ID | **SEQ ID No.** |
|---|---|---|---|
| Human | Homo sapiens | P38567 | **1** |
| Monkey | Nazalis larvatus | H2DJA7 | **2** |
| Chimpanzee | Pan troglodytes | A0A2J8QX33 | **3** |
| Bonobo | Pan paniscus | A0A2R8Z9G5 | **4** |
| Gorilla | Gorilla gorilla gorilla | G3QVL9 | **5** |
| Orangutan | Pongo abelii | A0A2J8U4U2 | **6** |
| Monkey | Symphalangus syndactylus | H8XWI5 | **7** |
| Monkey | Nomascus leucogenys | G1RNI9 | **8** |
| Bovine | Bos taurus | F1MTV1 | **9** |
| Ovine | Ovis ariens | W5NSU1 | **10** |

**Table 2. Amino acid sequences of animal PH20**

| Amino acid sequence | SEQ ID No. |
|---|---|
| MGVLKFKHIF FRSFVKSSGV SQIVFTFLLI PCCLTLNFRA PPVIPNVPFL WAWNAPSEFC LGKFDEPLDM SLFSFIGSPR INATGQGVTI FYVDRLGYYP YIDSITGVTV NGGIPQKISL QDHLDKAKKD ITFYMPVDNL GMAVIDWEEW RPTWARNWKP KDVYKNRSIE LVQQQNVQLS LTEATEKAKQ EFEKAGKDFL VETIKLGKLL RPNHLWGYYL FPDCYNHHYK KPGYNGSCFN VEIKRNDDLS WLWNESTALY PSIYLNTQQS PVAATLYVRN RVREAIRVSK IPDAKSPLPV FAYTRIVFTD QVLKFLSQDE LVYTFGETVA LGASGIVIWG TLSIMRSMKS CLLLDNYMET ILNPYIINVT LAAKMCSQVL CQEQGVCIRK NWNSSDYLHL NPDNFAIQLE KGGKFTVRGK PTLEDLEQFS EKFYCSCYST LSCKEKADVK DTDAVDVCIA DGVCIDAFLK PPMETEEPQI FYNASPSTLS ATMFIVSILF LIISSVASL | 1 |
| MGVIKFKHIF FRSFVKSSGV SQIVFTFLLI PCCLTLNFRA PPIIPNVPFL WAWNAPSEFC LGKFDEPLDM SLFSLIGSPR INVTGQGVTI FYVDRLGYYP YIDITTGVTV HGGIPQKVSL QDHLDKAKQD IIFYMPVDNL GMAVIDWEEW RPTWARNWKP KDVYKNRSIE LVQQQNVQLS LPEATDKAKQ EFEKAGKDFM LETIKLGKSL RPNHLWGYYL FPDCYNHHYK KPGYNGSCFD VEIKRNDDLS WLWNESTALY PSIYLNTQQS PVAATLYVRN RVQEAIRVSK IPDAKNPLPV FVYARLVFTD QVLKFLSRDE LVYTLGETVA LGASGIVIWG SLSIMRSMKS CLLLDNYMET ILNPYIINVT LAAKMCSQVL CQEQGVCIRK DWNSSDYLHL NPDNFAIQME KGGKFTVRGK PTLEDLEEFS EKFYCSCYTT LSCKEKADVK DTDAVDVCIA DGVCIDASLK PPVETEGSPP IFYNTSPSTL STTMFIVSIL FLIISSVVSL | 2 |
| MGVLKFKHIF FRSFVKSSGV SQIVFTFLLI PCCLTLNFRA PPVIPNVPFL WAWNAPSEFC LGKFDEPLDM SLFSFIGSPR INVTGQDVTI FYVDRLGYYP | 3 |
| YIDSITGVTV NGGIPQKISL QDHLDKAKKD ITFYMPVDNL GMAVIDWEEW RPTWARNWKP KDVYKNRSIE LVQQQNVQLS LTEATEKAKQ EFEKAGKDFL VETIKLGKLL RPNHLWGYYL FPDCYNHHYK KPGYNGSCFN VEIKRNDDLS WLWNESTALY PSIYLNTQQS PVAATLYVRN RVQEAIRVSK IPDAKSPLPV FVYTRIVFTD QVLKFLSQDE LVYTFGETVA LGASGIVIWG TLSIMRSMKS CLLLDNYMET ILNPYIINVT LAAKMCSQVL CQEQGVCIRK NWNSSDYLHL NPDNFAIQLE KGGKFTVRGK PTLEDLEQFS EKFYCSCYST LSCKEKADVK DTDAVDVCIA DGVCIDAFLK PPMETEESQI FYNASPSTLS ATMFIVSILF LIISSVASL | |
| MGVLKFKHIF FGSFVKSSGV SQIVFTFLLI PCCLTLNFRA PPVIPNVPFL WAWNAPSEFC LGKFDEPLDM SLFSFIGSPR INVTGQDVTI FYVDRLGYYP YIDSITGVTV NGGIPQKISL QDHLDKAKKD ITFYMPVDNL GMAVIDWEEW RPTWARNWKP KDIYKNRSIE LVQQQNVQLN LTEATEKAKQ EFEKAGKDFL VETIKLGKLL RPNHLWGYYL FPDCYNHHYK KPGYNGSCFN VEIKRNDDLS WLWNESTALY PSIYLNTQQS PVAATLYVRN RVREAIRVSK IPDAKSPLPV FVYTRIVFTD QVLKFLSQDE LVYTFGETVA LGASGIVIWG TLSIMRSMKS CLLLDNYMET ILNPYIINVT LAAKMCSQVL CQEQGVCIRK NWNSSDYLHL NPDNFAIQLE KGGKFTVRGK PTLEDLEQFS EKFYCSCYST LSCKEKADVK DTDAVDVCIA DGVCIDAFLK PPMETEESQI FYNASPSTLS ATMFIVSILF LIISSVASL | 4 |
| MGVLKFKHIF FRSFVKSSGV SQIVFTFLLI PCCLTLNFRA PPVIPNVPFL WAWNAPSKFC LGKFDEPLDM SLFSLIGSPR INITGQGVTI FYVDRLGYYP YIDSITGVTV NGGIPQKISL QDHLDKAKKD ITFYMPVDNL GMAVIDWEEW RPTWARNWKP KDVYKNRSIE LVQQQNVQLS LTEATEKAKQ EFEKAGKDFM VETIKLGKLL RPNHLWGYYL FPDCYNHHYK KPGYNGSCFN VEIKRNDDLS WLWNESTALY PSIYLNTQQS PVAATLYVRN RVQEAIRVSK IPDAKSPLPV FVYTRIVFTD QVLKFLSQDE LVYTFGETVA LGASGIVIWG TLSIMRSMKS CLLLDNYMET ILNPYIINVT LAAKMCSQVL CQEQGVCIRK DWNSSDYLHL NPDNFAIQLE KGGKFTVRGK PTLEDLEQFS EKFYCSCYST LSCKEKADIK DTDAVDVCIA DGVCIDAFLK PPMDTEESQI FYNASPSTLS ATMLIVSILF | 5 |
| LIISSVARL | |
| MGVLKFKHIF FRSFVKSSGV SQIVLTFLLI PCCLTLNFRA PPIIPNMPFL WAWNAPSEFC LGKFDEPLDM SLFSLIGSPR INVTGQAVTI FYVDRLGYYP YIDSITGVTV NGGIPQKISL QDHLDKAKKD ILFYMPVDNL GMAVIDWEEW RPTWARNWKP KDVYKNRSIE LVQQQNVQLN LTEATEKAKQ EFEKAGKDFM VETIKLGKLL RPNHLWGYYL FPDCYNHHYK KPGYNGSCFN VEIKRNDDLS WLWNESTALY PSIYLNTQQS PVAATLYVRN RVREAIRVSK IPDAKSPLPV FVYARIVFTD QVLKFLSQDE LVYTFGETVA LGASGIVIWG SLSIMRSMKS CLLLDNYMET ILNPYIINVT LAAKMCSQVL CQEQGVCIRK DWNSSDYLHL NPDNFAIQLE KGGKFTVRGK PTLEDLEQFS EKFYCSCYST LSCKEKADVK DTDAVDVCIA DGVCIDAFLK PPMETEESQI FYNASPSTLS ATMFIWRLEV WDQGISRMGF F | 6 |
| MGVLKFKHIF FRSFVKSNGV SQIVFTFLLI PRCLTLNFRA PPVIPNVPFL WAWNAPSEFC LGKFDEPLDM SLFSLIGSPR INVTGQGVTI FYVDRLGYYP YIDSITGVTV NGGIPQKISL QDHLDKAKQD ITFYMPVDNL GMAVIDWEEW RPTWARNWKP KDVYKNRSIE LVQQQNVQLN LTEATEKAKQ EFEKAGKDFM VETIKLGKLL RPNHLWGYYL FPDCYNHHYK KPGYNGSCFN VEIKRNDDLS WLWNESTALY PSIYLNTQQS PVAATLYVRN RVREAIRVSK IPDAKSPLPV FVYARIVFTD QVLKFLSRDE LVYTLGETVA LGASGIVIWG SLSIMRSMKS CLLLDNYMET ILNPYIINVT LAAKMCSQVL CQEQGVCIRK DWNSSDYLHL NPDNFAIQLE KGGKFTVRGK PTPEDLEQFS EKFYCSCYST LSCKEKADVK DTDAVDVCIA DGVCIDAFLK PPKETEESQI FYNASPSTLS ATMFIVSILF PIISSVVSL | 7 |
| MGVLKFKHIF FRSFVKSNGI SQIVFTFLLI PRCLTLNFRA PPVIPNVPFL WAWNAPSEFC LGKFDEPLDM SLFSLTGSPR INVTGQGVTI FYVDRLGYYP YIDSITGVTV NGGIPQKISL QDHLDKAKQD ITFYMPVDNL GMAVIDWEEW RPTWARNWKP KDVYKNRSIE LVQQQNVQLS LAEATEKAKQ EFEKAGKDFM VETIKLGKLL RPNHLWGYYL FPDCYNHHYK KPGYNGSCFN VEIKRNDDLS WLWNESTALY PSIYLNTQQS PVAATLYVRN RVREAIRVSK IPDAKSPLPV FVYARIVFTD QVLKFLSRDE LVYTLGETVA LGASGIVIWG SLSIVRSMKS | 8 |
| CLLLDNYMET ILNPYIINVT LAAKMCSQVL CQEQGVCIRK DWNSSDYLHL NPDNFAIQLE KGGKFTVRGK PTPEDLEQFS EKFYCSCYST LSCKEKADVK DTDAVDVCIA DGVCIDAFLK PPKETEESQI FYNASPSTLS ATMFIVSILF LIISSVVSL | |
| MRMLRRHHIS FRSFAGSSGT PQAVFTFLLL PCCLALDFRA PPLISNTSFL WAWNAPVERC VNRRFQLPPD LRLFSVKGSP QKSATGQFIT LFYADRLGYY PHIDEKTGKT VFGGIPQLGN LKSHLEKAKN DIAYYIPNDS VGLAVIDWEN WRPTWARNWK PKDVYRDESV ELVLQKNPQL SFPEASKIAK VDFETAGKSF MQETLKLGKL LRPNHLWGYY LFPDCYNHNH NQPTYNGNCP DVEKRRNDDL EWLWKESTAL FPSVYLNIRL KSTQNAALYV RNRVQEAIRL SKIASVESPL PVFVYARPVF TDGSSTYLSQ GDLVNSVGEI VSLGASGIIM WGSLNLSLSM QSCMNLGTYL NTTLNPYIIN VTLAAKMCSQ VLCHNEGVCT RKHWNSSDYL HLNPMNFAIQ TGEGGKYTVP GTVTLEDLQK FSDTFYCSCY ANIHCKKRVD IKNVHSVNVC MAEDICIDSP VKLQPSDHSS SQEASTTTFS SISPSTTTAT VSPCTPEKHS PECLKVRCSE VIPNVTQKAC QSVKLKNISY QSPIQNIKNQ TTY | 9 |
| KRMLRRRHIS FRSFVGASGT PQAALAFLLL PCWLALDFRA PPLISNTSFL WAWNAPAERC VKIFKLPPDL RLFSVKGSPQ KSATGQFITL FYADRLGYYP HIDEKTGNTV YGGIPQLGNL KNHLEKAKKD IAYYIPNDSV GLAVIDWENW RPTWARNWKP KDVYRDESVE LVLQKNPQLS FPEASKIAKV DFETAGKSFM QETLKLGKLL RPNHLWGYYL FPDCYNHNYN QPTYNGNCSD LEKRRNDDLD WLWKESTALF PSVYLNIKLK STPKAAFYVR NRVQEAIRLS KIASVESPLP VFVYHRPVFT DGSSTYLSQG DLVNSVGEIV ALGASGIIMW GSLNLSLTMQ SCMNLGNYLN TTLNPYIINV TLAAKMCSQV LCHDEGVCTR KQWNSSDYLH LNPMNFAIQT GKGGKYTVPG KVTLEDLQTF SDKFYCSCYA NINCKKRVDI KNVHSVNVCM AEDICIEGPV KLQPSDHSSS QNEASTTTVS SISPSTTATT VSPCTPEKQS PECLKVRCLE AIANVTQTGC QGVKWKNTSS QSSIQNIKNQ TTY | 10 |

Hyaluronidase of a human body is present in plasma or other organs, and includes Hyal1, Hyal2, Hyal3 and Hyal4, which are active at acidic pH, and PH20 which is expressed in a sperm acrosome and plays a role in fertilization.

A human PH20 polypeptide having the amino acid sequence of SEQ ID No. 1 consists of signal sequences of M1 to T35, hyaluronidase active sites of L36 to S490, and C-terminal GPI (Glycosyl-phosphatidyl inositol)-anchored sequences of A491 to L509, and a recombinant PH20 polypeptide converted to a soluble form lacking all of the C-terminal GPI-anchored sequences is used as a therapeutic or dispersing agent *in vivo.* In particular, it has been reported that enzyme activity is conserved while maintaining solubility even when some portion of the C-terminal amino acid sequence, i.e., any portion between I465 to L509, is cleaved. For a commercial product, the C-terminus ends at position 482. However, this case has maintained the sequence starting from L36 of the N-terminus, except for the signal sequence (WO 2010/077297A, etc.).

Recently, it is presented that a hyaluronidase variants having cleaved N-terminus which start with N37, F38, R39, A40, P41 or P42 as well as L36 are enzymatically active, and on this basis, hyaluronidase variant with good thermostability and enzymatic activity has been developed (WO 2020/022791A et al.).

A PH20 polypeptide of monkey (Nasalis larvatus) has 93.1% sequence identity to a human PH20 polypeptide. Therefore, based on the results of the multiple sequence analysis in FIG. 4, it can be expected that a PH20 polypeptide of monkey (Nasalis larvatus) with a C-terminus which ends atY483 is active and soluble PH20.

A PH20 polypeptide of bovine (Bos taurus) and a PH20 polypeptide of ovine (Ovis aries) share 90.6% sequence identity with each other, and 63.6% and 63.5% sequence identities with a human PH20 polypeptide, respectively. For a PH20 polypeptide of bovine (Bos taurus), Meyer et al. (1997) reported that a soluble polypeptide has a C-terminus which ends at H478. Based on the results of the multiple sequence analysis in FIG. 4, it can be expected that a PH20 polypeptide of ovine (Ovis aries) with a C-terminus which ends at H477 is active and soluble PH20.

The present invention provides an N-terminal and/or C-terminal cleaved recombinant PH20 polypeptide with improved productivity by further cleaving the N-terminus, that is, further deleting the amino acid residue from mature native PH20 polypeptide. The term "N-terminal and/or C-terminal cleaved recombinant PH20 polypeptide" used herein substantially refers to a PH20 variant.

The recombinant PH20 polypeptide according to the present invention is cleaved before an amino acid residue selected from the group consisting of N37 to P42 in wild-type PH20 having the amino acid sequence of SEQ ID No: 1, resulting in the deletion of (an) N-terminal amino acid residue(s).

Specifically, the recombinant PH20 polypeptide according to the present invention may include a sequence which is cleaved before an amino acid residue selected from the group consisting of N37 to P42, and starts at N37, F38, R39, A40, P41 or P42 of the amino acid sequence of SEQ ID No: 1. More specifically, the recombinant PH20 polypeptide according to the present invention may comprise a sequence starting at N37 or F38 of the amino acid sequence of SEQ ID No: 1.

The cleavage before an amino acid residue selected from the group consisting of N37 to P42 indicates that all of the amino acid residue(s) immediately preceding an amino acid residue selected from the group consisting of N37 to P42 at the N-terminus is(are) cleaved and deleted.

For example, the statement that the cleavage occurred before amino acid residue N37, F38, R39, A40, P41 or P42 means that, in the amino acid sequence of SEQ ID No. 1, residues 1 to 36 immediately before N37, residues 1 to 37 immediately before F38, residues 1 to 38 immediately before R39, residues 1 to 39 immediately before A40, residues 1 to 40 immediately before P41, and residues 1 to 41 immediately before P42 were cleaved and removed, respectively.

The recombinant PH20 polypeptide according to the present invention may further include a deletion of some amino acid residues at the C-terminus. Specifically, the recombinant PH20 polypeptide according to the present invention may be cleaved after an amino acid residue selected from the group consisting of F468 to Y482 of the amino acid sequence of SEQ ID No: 1, resulting in deletion of the amino acid residues at the C-terminus. More specifically, the recombinant PH20 polypeptide according to the present invention may comprise a sequence which ends at F468 or Y482 of the amino acid sequences of SEQ ID No: 1.

The cleavage after amino acid residues selected from the group consisting of F468 to Y482 of the amino acid sequences of SEQ ID No. 1, and the deletion of the amino acid residues at the C-terminus mean that all of the amino acid residues immediately after the amino acid residues selected from the group consisting of F468 to Y482 to the C-termini are cleaved and deleted. For example, the cleavage after residue F468 or Y482 means that all of the residues after F468 or Y482 of the amino acid sequences of SEQ ID No. 1 are cleaved and deleted.

In a specific embodiment, the recombinant PH20 polypeptide according to the present invention may be selected from the group consisting of:
(1) a PH20 polypeptide comprising an amino acid sequence starting from N37 and ending at Y482 (N37-Y482) of SEQ ID No. 1;
(2) a PH20 polypeptide comprising an amino acid sequence starting from N37 and ending at F468 (N37-F468) of SEQ ID No. 1;
(3) a PH20 polypeptide comprising an amino acid sequence starting from F38 and ending at Y482 (F38-Y482) of SEQ ID No. 1; or
(4) a PH20 polypeptide comprising an amino acid sequence starting from F38 and ending at F468 (F38-Y468) of SEQ ID No. 1.
(5) a PH20 polypeptide comprising an amino acid sequence starting from N37 and ending at L490 (N37-L490) of any one of SEQ ID Nos. 2 to 8;
(6) a PH20 polypeptide comprising an amino acid sequence starting from F38 and ending at L490 (F38-L490) of any one of SEQ ID Nos. 2 to 8;
(7) a PH20 polypeptide comprising an amino acid sequence starting from D37 and ending at H478 (D37-H478) of SEQ ID No. 9;
(8) a PH20 polypeptide comprising an amino acid sequence starting from F38 and ending at H478 (F38-H478) of SEQ ID No. 9;
(9) a PH20 polypeptide comprising an amino acid sequence starting from D37 and ending at H477 (D37-H477) of SEQ ID No. 10;
(10) a PH20 polypeptide comprising an amino acid sequence starting from F38 and ending at H477 (F38-H477) of SEQ ID No. 10;
(11) a PH20 polypeptide comprising an amino acid sequence starting from R39 and ending at H477 (R39-H477) of SEQ ID No. 10; and
(12) a PH20 polypeptide comprising an amino acid sequence starting from A40 and ending with H477 (A40-H477) of SEQ ID No. 10.

In another aspect, the present invention provides a composition for treating cancer comprising the recombinant PH20 polypeptide and a method of treating cancer using the composition.

The cancers are not particularly limited and include both solid and hematologic cancers. Examples of such cancers may be selected from the group consisting of, but are not limited to, skin cancer, including melanoma, liver cancer, hepatocellular carcinoma, stomach cancer, breast cancer, lung cancer, ovarian cancer, bronchial cancer, nasopharyngeal cancer, larynx cancer, pancreatic cancer, bladder cancer, colon cancer, cervical cancer, brain cancer, prostate cancer, bone cancer, thyroid cancer, parathyroid cancer, kidney cancer, esophageal cancer, biliary tract cancer, testicular cancer, rectal cancer, head and neck cancer, cervical spine cancer, ureteral cancer, osteosarcoma, neuroblastoma, fibrosarcoma, rhabdomyosarcoma, astrocytoma and glioma. Preferably, the cancers which can be treated with the composition of the present invention may be selected from the group consisting of, but are not limited to, colon cancer, breast cancer, lung cancer and kidney cancer.

The composition may be a pharmaceutical composition. The pharmaceutical composition may further comprise a pharmaceutically acceptable carrier, wherein the carrier is conventionally used in the formulation of drugs, and may be at least one selected from the group consisting of, but not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil. The pharmaceutical composition may further comprise at least one selected from the group consisting of diluents, excipients, lubricants, humectants, sweeteners, flavoring agents, emulsifiers, suspensions and preservatives, which are commonly used in the preparation of pharmaceutical compositions.

The pharmaceutical composition can be administered orally or parenterally. If parenterally administered, it can be administered by intravenous injection, subcutaneous injection, intramuscular injection, intraperitoneal injection, endothelial administration, topical administration, intranasal administration, intrapulmonary administration or rectal administration. Proteins or peptides are digestible when administered orally, an oral composition may be formulated to coat the active agent or to protect it from degradation in the stomach. Additionally, the composition may be administered by any device that allows the active agent to be transported to the target cell.

The pharmaceutical composition may be in the form of a solution, suspension, syrup or emulsion in an oil or aqueous medium, or can be formulated in the form of an extract, powder, granule, tablet or capsule, and may further comprise a dispersing agent or a stabilizing agent for formulation.

In particular, the composition for the treatment of cancer according to the present invention is characterized for use in combination therapy with other anticancer agents.

Preferred anticancer agents for use in combination therapy include, but are not limited to, chemotherapeutic agents, antibody-type anticancer agents, RNAi or cellular therapies.

Anti-cancer agents that can be used in combination therapy are, but are not limited to, immuno-anticancer agents, and more preferably immune checkpoint inhibitors.

In another aspect, the present invention relates to a nucleic acid encoding the recombinant PH20 polypeptide having a deletion of amino acid residue at the N-terminus and/or C-terminus of mature animal wild-type PH20 according to the present invention.

The nucleic acid used herein may be present in cells or cell lysates, or may be present in the partially purified or substantially pure form. A nucleic acid is "isolated" or "substantially pure" when it is purified from other cellular components or other contaminants, such as a nucleic acid or protein from other cells, by standard techniques including alkaline/SDS treatment, CsCl banding, column chromatography, agarose gel electrophoresis, and other techniques commonly known in the art. The nucleic acid of the present invention may be, for example, DNA or RNA.

In another aspect, the present invention relates to a vector comprising the nucleic acid, in particular, a recombinant expression vector. For the expression of the recombinant PH20 recombinant polypeptide according to the invention, a DNA encoding a PH20 recombinant polypeptide can be obtained by a standard molecular biology technique (e.g. PCR amplification or cDNA cloning using a hybridoma expressing a PH20 recombinant polypeptide), and the DNA can be "operably linked" to a transcriptional and translational control sequence and inserted into an expression vector.

The term "operably coupled" used herein can mean that a gene encoding a PH20 recombinant polypeptide is ligated into the vector such that the transcriptional and translational control sequence in the vector has the intended function of regulating transcription and translation of the gene encoding a PH20 recombinant polypeptide. The expression vector and the expression control sequence are selected to be compatible with the host cell for expression to be used. The gene encoding a PH20 recombinant polypeptide is inserted into the expression vector by a standard method (e.g., ligation of a gene fragment encoding a PH20 recombinant polypeptide and a complementary restriction enzyme site on a vector, or blunt end ligation if no restriction enzyme site is present).

Further, the recombinant expression vector has a regulatory sequence that controls expression of a gene encoding a PH20 recombinant polypeptide in a host cell. The "regulatory sequence" may include promoters, enhancers, or other expression control elements (e.g., polyadenylation signals) that control transcription or translation of a gene encoding a PH20 recombinant polypeptide. A person skilled in the art can recognize that design of an expression vector may vary by selecting a different control sequence depending on factors such as the type of host cell to be transformed, or the expression level of protein.

In another aspect, the invention relates to a host cell comprising the nucleic acid or the vector. The host cell according to the present invention is preferably selected from the group consisting of, but not limited to, animal cells, plant cells, yeast, E. coli and insect cells.

Specifically, the host cell according to the present invention may be a prokaryotic cell such as Escherichia coli, Bacillus subtilis, Streptomyces sp., Pseudomonas sp., Proteus mirabilis or Staphylococcus sp. It can also be fungi such as Aspergillus sp., yeasts such as Pichia pastoris, Saccharomyces cerevisiae, Schizosaccharomyces sp. or Neurospora crassa, other lower eukaryotic cells, or eukaryotic cells such as cells of higher eukaryotes such as insects.

Furthermore, the host cell according to the present invention can be derived from plants or mammals. Monkey kidney cells 7 (COS7), NSO cells, SP2/0, Chinese hamster ovary (CHO) cells, W138, baby hamster kidney (BHK) cells, MDCK, myeloma cell lines, HuT 78 cells or HEK293 cells can be preferably used, but are not limited thereto. Particularly preferably, CHO cells can be used.

The nucleic acid or vector is transfected into a host cell. For "transfection," a number of different techniques commonly used to introduce an exogenous nucleic acid (DNA or RNA) into a prokaryotic or eukaryotic host cell can be used, such as electrophoresis, calcium phosphate precipitation, DEAE-dextran transfection or lipofection. Various expression host/vector combinations may be used to express the PH20 recombinant polypeptide according to the present invention. Suitable expression vectors for a eukaryotic host include, but are not limited to, expression control sequences derived from SV40, bovine papillomaviruses, adenoviruses, adeno-associated viruses, cytomegaloviruses and retroviruses. An expression vector that can be used in a bacterial host includes bacterial plasmids obtained from Escherichia coli, such as pET, pRSET, pBluescript, pGEX2T, pUC vectors, col E1, pCR1, pBR322, pMB9 or derivatives thereof; plasmids with a broader host range, such as RP4; phage DNA which can be exemplified by a very wide variety of phage lambda derivatives such as λgt10 and λgt11 or NM989; and other DNA phages, such as M13 or filamentous single-stranded DNA phage. An expression vector useful for yeast cells is a 2°C plasmid and derivatives thereof. A vector useful for insect cells is pVL941.

In another aspect, the present invention relates to a method of producing the recombinant PH20 recombinant polypeptide according to the present invention, comprising the step of culturing a host cell to express the recombinant PH20 recombinant polypeptide according to the present invention.

When a recombinant expression vector capable of expressing the PH20 recombinant polypeptide is introduced into a mammalian host cell, the PH20 recombinant polypeptide may be produced by culturing the host cell for a period of time sufficient to be expressed in the host cell, or more preferably for a period of time sufficient to allow secretion of the PH20 recombinant polypeptide into the culture medium in which the host cell is cultured.

In some cases, the expressed PH20 recombinant polypeptide can be isolated from the host cell and purified to homogeneity. The isolation or purification of the PH20 recombinant polypeptide can be carried out by any isolation or purification methods commonly used for proteins, such as chromatography. The chromatography may be, for example, but are not limited to, one or more combinations selected from affinity chromatography, ion exchange chromatography or hydrophobic chromatography. In addition to the chromatography, filtration, ultrafiltration, salting-out or dialysis can be used in combination.

Hereinafter, Examples are presented to further describe the present invention. The following Examples are provided only to illustrate the present invention, and it is obvious to a person skilled in the art that the scope of the present invention is not limited to these Examples.

### Example 1. Cloning of N-terminal and/or C-terminal Cleaved Recombinant PH20 Hyaluronidase

For expression of PH20 polypeptide, the sequence encoding amino acids L36 to S509 of wild-type human PH20 polypeptide, the DNA sequence encoding amino acids M1 to L510 of PH20 polypeptide (Uniprot ID: H2DJA7) from monkey (Nasalis larvatus), the DNA sequence encoding amino acids L36 to H478 of PH20 polypeptide (Uniprot ID: F1MTV1) from bovine (Bos taurus), and the DNA sequence encoding amino acids L36 to H477 of PH20 polypeptide (Uniprot ID: W5NSU1) from ovine (Ovis aries) were synthesized by Genscript (Korea).

Each synthesized PH20 polypeptide gene was amplified by polymerase chain reaction (hereinafter, "PCR") and inserted into the XhoI or NotI restriction enzyme site of the pcDNA3.4-TOPO vector. For expression in ExpiCHO cells, a human serum albumin signal peptide was used. For protein purification using a HisTrap column, the DNA sequence of His-Tag was positioned at the 3'-end of the PH20 cDNA. An N-terminal and/or C-terminal cleaved PH20 polypeptide was subjected to a PCR, and amino acid substitutions were confirmed by DNA sequencing.

In order to produce an N-terminal cleaved variant of human PH20 polypeptide, a plasmid containing L36-Y482 was prepared and used as a template to produce four variants in which amino acids were sequentially removed one by one from the N-terminus, and for each variant, four variants in which the C-terminus ends at F468.

To produce an N-terminal cleaved variant of monkey (Nasalis larvatus) PH20 polypeptide, a plasmid containing L36 to Y483 was prepared and used as a template to produce five variants in which amino acids were sequentially removed one by one from the N-terminus.

To produce an N-terminal cleaved variant of bovine (Bos Taurus) PH20 polypeptide, a plasmid containing L36 to H478 was prepared and used as a template to produce five variants in which amino acids were sequentially removed one by one from the N-terminus.

To produce an N-terminal cleaved variant of ovine (Ovis aries) PH20 polypeptide, a plasmid containing L36 to H477 was prepared and used as a template to produce five variants in which amino acids were sequentially removed one by one from the N-terminus.

### Example 2. Expression and Purification of N-terminal and/or C-terminal Cleaved Recombinant PH20 Hyaluronidase

Expression of an N-terminal and/or C-terminal cleaved PH20 polypeptide was carried out by using an ExpiCHO expression system. When the cell density of ExpiCHO cell reached 6x10⁶ cells/mL, a plasmid containing the cDNA of the N-terminal and C-terminal cleaved PH20 polypeptide inserted into the pcDNA3.4-TOPO vector was transfected into ExpiCHO cells using an ExpiFectamine CHO reagent. As a cell culture medium, an ExpiCHO expression medium (100 to 500 mL) was used. After transfection, ExpiCHO cells were shaken and cultured at 130 rpm for a total of 6 days, during which the cells were incubated at 37°C for 1 day and further incubated at a lower temperature of 32°C for 5 days. After completion of incubation, cell supernatants were collected by centrifugation at 10,000 rpm for 30 minutes.

A PH20 polypeptide with a His-tag at the C-terminus produced from ExpiCHO cells was purified by the two-step column chromatography using an AKTA prime equipment or similar equipment (GE Healthcare). For human PH20 and monkey PH20, Q Sepharose, which is an anion exchange chromatography, was used because the pI is close to 6. For bovine or ovine PH20 polypeptide, the pI is above 8, so the first purification was performed using a Capto S column, which is a cation exchange chromatography, and the second purification for each protein was performed using a HisTrap HP column, which is a His-Tag-friendly chromatography.

For purification of protein by using a Q Sepharose column, buffer A (20 mM sodium phosphate, pH 7.5) and buffer B (20 mM sodium phosphate, pH 7.5, 0.5 M NaCl) were prepared. The pH and conductivity of the culture medium were adjusted to be the same as Buffer A, and the culture medium was filtered through a membrane having a pore size of 0.22 µm. Proteins were bound to a Q Sepharose column, 5CV of buffer A was flowed to remove non-specifically bound proteins, and 5CV of buffer B was flowed with 0 to 100% of concentration gradient, so as to elute proteins.

For purification of protein using a Capto S column, Buffer A (20 mM sodium phosphate, 15 mM NaCl, pH 6.0) and Buffer B (20 mM sodium phosphate, 500 mM NaCl, pH 6.0) were prepared. The pH and conductivity of the culture medium were adjusted to be the same as Buffer A, and the culture medium was filtered through a membrane having a pore size of 0.22 µm. Proteins were then bound to a Capto S column, and 3 column volumes (CV) of Buffer A was flowed to remove non-specifically bound proteins. 4CV of Buffer B was sequentially flowed to elute the target protein.

For purification of protein by using a HisTrap HP column, Buffer A (20 mM sodium phosphate, 500 mM NaCl, pH 7.5) and Buffer B (20 mM sodium phosphate, 500 mM NaCl, 500 mM Imidazole, pH 7.5) were prepared. After the protein sample was bound to the HisTrap HP column, 7 CV of 7% Buffer B was flowed to remove non-specifically bound proteins, and 3 CV of 40% Buffer B was flowed to elute the target protein. The column eluate was dialyzed using a dialysis buffer (20 mM sodium phosphate, 100 mM NaCl, pH 7.0).

### Example 3. N-terminal Sequencing of N-terminal and/or C-terminal Cleaved Recombinant PH20 Hyaluronidase

The purified N-terminal and/or C-terminal cleaved PH20 polypeptide was loaded in an amount of 10 µg per lane of a 7.5% SDS-PAGE gel and electrophoresis (150 V, 1 hour) was performed. The gel containing the electrophoresis-deployed proteins was then introduced in a blotting kit with a PVDF Membrane and transferred at 100V for 90 minutes, and the transfer was confirmed by Ponceau S staining. Finally, the samples obtained by incising each protein band were sequenced for the 5 amino acids from N-terminus using a PPSQ-53A Protein sequencer (Shimadzu, Japan).

As a result of the N-terminal sequencing, other PH20 polypeptides were confirmed to have the expected sequence, but in some human PH20 polypeptides having N37-Y482 or N37-F468, aspartate was found rather than asparagine as an amino acid at the N-terminus, resulting in de-amidation. Based on this result and the multiple sequence analysis of FIG. 4 showing that the aspartate were found as the corresponding amino acid in bovine and ovine, it can be inferred that aspartate as an amino acid at this position plays a role in stabilizing the structure of protein.

### Example 4. Measurement of Activity of Recombinant N-terminal and/or C-terminal Cleaved PH20 Hyaluronidase Activity

The activity of hyaluronidase was measured by a turbidimetric assay, which measures turbidity, caused by precipitates produced when a hyaluronic acid is mixed with albumin (BSA), in absorbance. When a hyaluronic acid is hydrolyzed by a PH20 polypeptide, the turbidity/absorbance of the precipitates produced by mixing hyaluronic acid and albumin decreases. This assay is typically performed at pH 5.3 and as follows. Hyaluronidase standards having known activities (units) were diluted to 6, 8, 10, 12, 15 and 20 units/mL and prepared in each test tube. Purified protein samples were diluted in buffer (20 mM sodium phosphate, pH 5.3, 77 mM sodium chloride, and 0.01% (w/v) bovine serum albumin) by adjusting the dilution factor to fit within the range of the standard curve. 50 µl of the diluted sample was dispensed into each well of a 96-well plate and subjected to a warming reaction at 37°C for 10 min. 50 µl of 0.06% hyaluronic acid was additionally dispensed into each well. The 0.06% hyaluronic acid was in the state of being dissolved in 300 mM of sodium phosphate buffer having a pH of 5.3. The samples and 0.06% hyaluronic acid were reacted at 37°C for 45 minutes. After the reaction, 40 µl of the enzyme-substrate reaction solution was dispensed into 200 µl of acidic albumin solution and left at room temperature for 19 min. The absorbance was then measured at 600 nm by using a spectrophotometer. The acidic albumin solution is composed of 0.1% albumin (BSA) dissolved in a buffer having a pH of 3.75 comprising 24 mM of sodium acetate and 79 mM of acetic acid. The absorbance values of the measured samples were converted to activities by a standard curve using an active standard.

When performing the above steps at pH 7.0, the protein sample buffer was 20 mM of sodium phosphate having a pH of 7.0, 77 mM of sodium chloride, and 0.01% (w/v) bovine serum albumin, and for the 0.06% hyaluronic acid aqueous solution, hyaluronic acid was dissolved in 20 mM of sodium phosphate buffer having a pH of 7.0, and 70 mM of sodium chloride.

These activity measurements can also be made in culture media. In this case, since values below 300 units/mL are unreliable, the limit of quantification (LOQ) was set to 300 units/mL, and values below this were labeled as inactive, 0. Also, in the case of activity measurement using purified protein samples, the limit of quantification (LOQ) was set to 15 units/*µ*g.

The N-terminus was adjusted to L36, N37, F38, R39 or A40 excluding the signal sequence of M1 to T35 of wild-type PH20 polypeptide of SEQ ID No. 1 and the C-terminus was selected to F468 or Y482, a total of 10 N-terminal and/or C-terminal cleaved PH20 polypeptides were produced by the method of Example 1. Each of these clones was purified in the culture medium obtained by transient transfection culture of animal cells, and the enzyme activities at pH 5.3 and pH 7.0 were compared by the method of Example 2.

As shown in FIG. 1, from the result comparing human PH20 activities in each culture medium, surprisingly, the activity of N37-Y482 in the culture medium was more than 3 times higher than the activity in the culture medium of mature L36-Y482 having a wild-type N-terminus, and the human PH20 activity of F38-Y482 in which two amino acid residues were additionally deleted from the N-terminus in the culture medium was also more than 2 times higher than the activity in the culture of mature L36-Y482 having a wild-type N-terminus.

However, R39-Y482, which lacks three N-terminal amino acid residues, resulted in little expression of enzyme, and A40-Y382 which lacks of four N-terminal amino acid residues also resulted in little expression of enzyme in a culture.

In conclusion, for human hyaluronidase PH20, it was found that variants lacking one or two N-terminal amino acid residues in mature human hyaluronidase PH20 showed significantly increased expression in a culture medium. In particular, considering the industrial availability of human hyaluronidase PH20 for an injection, it is shown that industrially and economically outstanding variants are obtainable by producing a variant enzyme in which one or two N-terminal amino acids are cleaved.

Furthermore, as shown in FIGs. 2 and 3, there is little difference between the specific activities of L36-Y482, N37-Y482 and F38-Y482, confirming that the differences in activity of human hyaluronidase PH20 in FIG. 1 are due to the differences in productivity. Furthermore, among PH20 variants with 14 C-terminal amino acids shorter than the protein whose C-terminus ends at Y482, only N37-F468 with one N-terminal amino acid deleted was active in the culture medium.

In particular, FIG. 2 shows the specific activities of each purified polypeptide at pH 5.3, confirming that there was little difference between L36-Y482, N37-Y482 and F38-Y482 when the C-terminus was Y482, but when the C-terminus was F468, N37-F468 and F38-F468 had 1.5 to 2 times higher specific activity than L36-F468. The same patterns were shown in the specific activities at pH 7.0.

That is, based on the results shown in FIGs. 2 and 3, shorter variant of commercially used PH20, L36-F468, in which C-terminal amino acids were further cleaved to end at position 468 was barely expressed in animal cells. As a result of purifying the very small amount of L36-F468 variant expressed therein and measuring the specific activity thereof, the specific activity of enzyme was approximately 60% compared to L36-Y482. Based on these findings, the hyaluronidase activity or productivity of a PH20 polypeptide significantly increases when one or two additional N-terminal amino acid residues are deleted from mature human hyaluronidase PH20.

In conclusion, although the specific activity of human hyaluronidase PH20 variant enzyme consisting of L36-F468 was not significantly reduced compared to the L36-Y482 PH20 variant enzyme, its expression in a recombinant cell was extremely low, making it unsuitable for industrial use.

On the other hand, for the N37-F468 variant, the expression in cells significantly increased, which is similar to the expression of the L36-Y482 variant, but the expression of N37-F468 in cells, which has one N-terminal amino acid further deleted, dramatically increased by at least 100-fold compared to L36-F468.

In view of the molecular modeling of PH20 and the experimental results of the present invention, the interaction between the amino acids at the N-terminus and the amino acids at the C-terminus of PH20 affects the stability, folding rate, etc. during transcription and translocation of recombinant PH20 protein when it is expressed in cells, and furthermore, greatly affects the expression of recombinant protein secreted from the cells. This significantly affects the productivity of PH20 and variants thereof. These results can be applied not only to human PH20, but also to mammalian-derived PH20, such as bovine or ovine, and further, are expected to be applied to other PH20s with structural or physicochemical properties similar to human PH20.

Furthermore, it was found that these properties were maintained in mammalian PH20s having similar sequence structure to a human PH20 polypeptide and the same biofunction, particularly the monkey (Nasalis larvatus), bovine (Bos taurus), and ovine (Ovis aries) PH20s listed in Table 1, as shown in FIGs. 5 and 6. In particular, a ovine PH20 polypeptide was found to be highly expressed when the N-terminus began with A40.

In summary, the results of the present invention show that for a mature PH20 protein, particularly an animal PH20 such as a human PH20, the removal of an additional one to four, preferably one or two, amino acid residues at the N-terminus can dramatically increase the expression rate in a recombinant cell, thereby increasing protein productivity.

For a human PH20, when the C-terminus was terminated at Y482, PH20 with some additional N-terminal amino acid residues removed from the mature PH20 showed a significant increase in expression with no significant change in the intrinsic activity of hyaluronidase, and when the C-terminus ended at F468, a variant with one or two additional N-terminal amino acid residues removed from the mature PH20 rather showed an increase in the specific activity of the enzyme compared to a mature protein having a complete N-terminus. While there have been attempts to increase the specific activity of protein by substituting different amino acids at specific sites in the amino acid sequence of human PH20, whereas no case has been reported to significantly increase its expression in cells by removing amino acids at the N-terminus while maintaining or increasing its activity. It is predicted that the N-terminal deleted variants are advantageous in terms of immunogenicity over PH20 in which (an) amino acid(s) is(are) replaced at (a) specific site(s), even after prolonged administration in a human body.

There have been reports showing that the expression is increased when the N-terminus is cleaved in a mature protein such as studies about human alpha 1-antitrypsin (H. Johansen et al., Mol. Biol. Med. 1987, 4:291-305) and human papillomavirus L1 protein (M. Wei et al., Emerging Microbes & Infections, 2018, 7:160), but it is difficult to find similarities to each other in terms of location of amino acids at the N-terminal site, number of truncation, etc. For example, in human alpha 1-antitrypsin, deletion of 5 to 10 N-terminal amino acids increased expression in cells but did not change the activity of the enzyme, and in human papillomavirus L1 protein, cleavage of 10 or 15 N-terminal amino acids did not change the overall expression of the protein but increased the solubility of the protein in some cases. In particular, no such attempts have been made for hyaluronidase such as PH20, and no cases have been found in which cleavage of one or two N-terminal amino acids dramatically increases expression and activity as in the present invention.

The three-dimensional crystal structure of human PH20 is not yet known. However, from the prediction of three-dimensional structure of PH20 using a program, it is predicted that while the amino acids F38, R39 and Y434 are located close to the asparagine side chain at position 37 at the N-terminus of PH20 and have appropriate charge interactions therebetween, the amino acid Leu36 in a mature protein does not form any specific bonds with the surrounding amino acids, and hydrophobic amino acids are exposed to an aqueous solution, thereby adversely affecting the speed of bonding or stability, and further, expression of the protein.

In a mature wild-type animal PH20 protein, a variant with amino acid substitution that does not significantly alter the three-dimensional structure of the protein may show the same results as the present invention. Since the effects thereof were also confirmed in bovine and ovine PH20s with 63.6% and 63.5% identity to human PH20, respectively, and primate PH20 with more than 93.1% identity to human PH20, the same results can be predicted for PH20 with 60%, 70%, 80%, 90%, 91%, 92%, 93%, 94%, 95%, 96%, 97%, 98% or 99% identity to human PH20, within the range that the three-dimensional structure of protein is not significantly modified.

### Example 5. Preparation of N-terminal and/or C-terminal Cleaved Recombinant PH20 Hyaluronidases Having Different Signal Peptides and Measurement of Activities

Nine variants replaced with a single peptide of human PH20, human serum hormone, or Ig kappa were prepared to identify changes in signal peptide. The sequences of the signal peptides are listed in Table 2 below.

In the previous examples, the signal peptide was from human serum albumin (HSA), and it was tested whether other signal peptides listed in Table 2 also achieve the same results.

Except for applying the signal peptides in Table 2, L36-Y482, N37-Y482 and F38-Y482 of recombinant human PH20 were prepared as in Examples 1 and 2, and the activity assay as in Example 4 was carried out, and the results are shown in FIG. 7.

As shown in FIG. 7, even when signal peptides of human PH20 or Ig kappa were used, productivity changes depending on N-terminal deletion of mature PH20 tended to be similar with case using signal peptide of human serum hormone. In this regard, it was confirmed that the increase in expression and productivity in a mature PH20 with further deletion of N-terminal amino acid is less related to the type of signal peptide.

### Industrial Availability

The N-terminal and/or C-terminal cleaved PH20 polypeptide with improved enzymatic activity and productivity presented herein exhibits superior expression and higher enzymatic activity compared to conventional recombinant PH20 polypeptides, which may result in reduction in therapeutic costs due to lower production costs for industrial use.

The foregoing has described in detail certain aspects of the present invention. It is apparent to a person skilled in the art that these specific examples are merely preferred embodiments and are not intended to limit the scope of the present invention.

Accordingly, the substantial scope of the invention is defined by the appended claims and equivalents thereof.

### References

1. H. Johansen, J. Sultiphong, G. Sathe, P. Jacobs, A. Cravador, A. Bollen, M. Rosenberg, and A. Shatzman, "High-level production of fully active human alpha 1-antitrypsin in Escherichia coli." Mol. Biol. Med. (1987) 4:291-305,
**2.** J.H. Dunham, R.C. Meyer, E.L. Garcia, and R.A. Hall, "GPR37 Surface Expression Enhancement via N-Terminal Truncation or Protein-Protein Interactions", Biochemistry (2009) 48:10286-10297
3. M. Wei, D. Wang, Z. Li, S. Song, X. Kong, X. Mo, Y. Yang, M. He, Z. Li, B. Huang, Z. Lin, H. Pan, Q. Zheng, H. Yu, Y. Gu, J. Zhang, S. Li and N. Xia, "N-terminal truncations on L1 proteins of human papillomaviruses promote their soluble expression in Escherichia coli and self-assembly in vitro", Emerging Microbes & Infections (2018) 7:160
4. M. F. Meyer, G. Kreil, and H. Aschauer, "The soluble hyaluronidase from bull testes is a fragment of the membrane-bound PH-20 enzyme", FEBS letter (1997) 413:385-388

## Claims

1. A recombinant PH20 polypeptide in which 1 to 7 amino acid residues are deleted at the N-terminus of mature animal wild-type PH20.

2. The recombinant PH20 polypeptide according to claim 1, **characterized by** being selected from the group consisting of:
(a) a recombinant PH20 polypeptide with (an) N-terminal amino acid residue(s) deleted by cleavage before an amino acid residue selected from the group consisting of N37 to P42 in a human wild-type PH20 having the amino acid sequence of SEQ ID NO. 1;
(b) a recombinant PH20 polypeptide with (an) N-terminal amino acid residue(s) deleted by cleavage before an amino acid residue selected from the group consisting of N37 to P42 in a primate wild-type PH20 having the amino acid sequence of any one of SEQ ID NOs. 2 to 8;
(c) a recombinant PH20 polypeptide with (an) N-terminal amino acid residue(s) deleted by cleavage before an amino acid residue selected from the group consisting of D37 to P42 in a bovine wild-type PH20 having the amino acid sequence of SEQ ID NO. 9; and
(d) a recombinant PH20 polypeptide with (an) N-terminal amino acid residue(s) deleted by cleavage before an amino acid residue selected from the group consisting of D37 to P42 in a ovine wild-type PH20 having the amino acid sequence of SEQ ID NO. 10.

3. The recombinant PH20 polypeptide according to claim 1, comprising a sequence starting at N37, F38, R39, A40, P41 or P42 in the amino acid sequence of SEQ ID NO. 1.

4. The recombinant PH20 polypeptide according to claim 1, comprising a sequence starting at N37 or F38 in the amino acid sequence of SEQ ID NO. 1.

5. The recombinant PH20 polypeptide according to claim 1, wherein some amino acid residues at the C-terminus are further deleted.

6. The recombinant PH20 polypeptide according to claim 5, **characterized in that** (a) C-terminal amino acid residue(s) is(are) deleted by cleavage after an amino acid residue selected from the group consisting of F468 to Y482 in the amino acid sequence of SEQ ID NO. 1.

7. The recombinant PH20 polypeptide according to claim 6, comprising a sequence which ends at F468 or Y482 in the amino acid sequence of SEQ ID NO. 1.

8. The recombinant PH20 polypeptide according to claim 1, **characterized by** being selected from the group consisting of:
(1) a PH20 polypeptide comprising an amino acid sequence starting from N37 and ending at Y482 (N37-Y482) of SEQ ID No. 1;
(2) a PH20 polypeptide comprising an amino acid sequence starting from N37 and ending at F468 (N37-F468) of SEQ ID No. 1;
(3) a PH20 polypeptide comprising an amino acid sequence starting from F38 and ending at Y482 (F38-Y482) of SEQ ID No. 1; or
(4) a PH20 polypeptide comprising an amino acid sequence starting from F38 and ending at F468 (F38-Y468) of SEQ ID No. 1.
(5) a PH20 polypeptide comprising an amino acid sequence starting from N37 and ending at L490 (N37-L490) of any one of SEQ ID Nos. 2 to 8;
(6) a PH20 polypeptide comprising an amino acid sequence starting from F38 and ending at L490 (F38-L490) of any one of SEQ ID Nos. 2 to 8;
(7) a PH20 polypeptide comprising an amino acid sequence starting from D37 and ending at H478 (D37-H478) of SEQ ID No. 9;
(8) a PH20 polypeptide comprising an amino acid sequence starting from F38 and ending at H478 (F38-H478) of SEQ ID No. 9;
(9) a PH20 polypeptide comprising an amino acid sequence starting from D37 and ending at H477 (D37-H477) of SEQ ID No. 10;
(10) a PH20 polypeptide comprising an amino acid sequence starting from F38 and ending at H477 (F38-H477) of SEQ ID No. 10;
(11) a PH20 polypeptide comprising an amino acid sequence starting from R39 and ending at H477 (R39-H477) of SEQ ID No. 10; and
(12) a PH20 polypeptide comprising an amino acid sequence starting from A40 and ending at H477 (A40-H477) of SEQ ID No. 10.

9. A nucleic acid encoding the recombinant PH20 polypeptide according to any one of claims 1 to 8.

10. A recombinant expression vector comprising a nucleic acid according to claim 9.

11. A host cell transfected with a recombinant expression vector according to claim 10.

12. The host cell according to claim 11, **characterized by** being selected from the group consisting of animal cells, plant cells, yeast, E. coli, and insect cells.

13. A method of producing a recombinant PH20 polypeptide, comprising the step of culturing the host cell according to claim 12.
